# EUROPÄISCHE PATENTSCHRIFT

(11) **EP 1 106 601 B1**
(45) Veröffentlichungstag und Bekanntmachung des Hinweises auf die Patenterteilung: **25.02.2004**
(21) Anmeldenummer: 00124800.4
(22) Anmeldetag: 14.11.2000
(51) Int. Cl.: C07C 209/26, C07C 211/06

(54) **Verfahren zur Herstellung von Monoisopropylamin**
Process for the preparation of monoisopropylamine
Procédé pour la préparation de la mono-isopropylamine

(30) Priorität: 06.12.1999 DE 19958701
(43) Veröffentlichungstag der Anmeldung: 13.06.2001
(73) Patentinhaber: BASF AKTIENGESELLSCHAFT, 67056 Ludwigshafen (DE)
(72) Erfinder: Nouwen, Jan, Dr., 64653 Lorsch (DE); Käshammer, Stefan, Dr., 67105 Schifferstadt (DE); Höhn, Arthur, Dr., 67281 Kirchheim (DE); Funke, Frank, Dr., 67227 Frankenthal (DE); Melder, Johann-Peter, Dr., 67459 Böhl-Iggelheim (DE); Gutschoven, Frank, 2018 Antwerpen (BE); Buskens, Philip, Dr., 2320 Hoogstraten (BE)

(56) Entgegenhaltungen:
- EP-A- 0 382 049
- EP-A- 0 514 692
- EP-A- 0 696 572
- EP-A- 0 963 975
- EP-A- 1 035 106
- DE-A- 1 817 691
- DE-A- 1 953 263
- US-A- 5 952 529

## Beschreibung

Die vorliegende Erfindung betrifft ein Verfahren zur Herstellung von Monoisopropylamin (MIPA; (CH₃)₂CHNH₂) durch Umsetzung von Aceton bei erhöhter Temperatur und erhöhtem Druck mit Ammoniak und mit Wasserstoff in Gegenwart eines Katalysators.

MIPA ist ein wichtiges organisches Zwischenprodukt, dass unter anderem als Vorprodukt zur Herstellung von Pflanzenschutzmittel benötigt wird.

MIPA wird in der Technik durch aminierende Hydrierung von Isopropanol oder Aceton mit Ammoniak an Katalysatoren hergestellt.

DE-A-1 543 354 offenbart die Synthese von MIPA durch aminierende Hydrierung von Aceton in Gegenwart von Kobaltkatalysatoren.

DT-A-1803 083, DT-A-1817 691, FR-A-159 0871, DE-A-18 03 083 und HU-A-47456 beschreiben die Herstellung von MIPA durch aminierende Hydrierung von Aceton in Gegenwart von Katalysatoren vom Raney-Nickel-Typ.

DL-A-75086, DE-A-17 93 220, GB-A-1218 454, CN-A-1130 621, CS-A-185 962 und CS-A-239 445 betreffen die Herstellung von MIPA durch aminierende Hydrierung von Aceton in Gegenwart von Nickel/ Aluminiumoxid-Katalysatoren.

DE-A-22 19 475 (loc. cit., Beispiele 10 bis 12) beschreibt die Synthese von MIPA aus Aceton in Gegenwart eines Nickel/Chrom/Kieselgur-Katalysators.

EP-A-284 398 und CN-A-111 0629 betreffen mit Ru bzw. mit Cr und Cu dotierte Nickelkatalysatoren zur Herstellung von MIPA aus Aceton.

Aus der älteren deutschen Anmeldung Nr. 19910960.5 vom 12.03.99 sind aus Nickel-, Kupfer-, Molybdän- und Zirkoniumdioxid bestehende Katalysatoren zur Herstellung von MIPA durch katalytischen Aminierung von Aceton in Gegenwart von Wasserstoff bekannt (loc. cit., Beispiel 1). Die Selektivität dieser Umsetzung beträgt nur 82,3 bis 95,2 %.

Die ältere deutsche Anmeldung Nr. 19859776.2 vom 23.12.98 beschreibt die Herstellung von MIPA aus Aceton und Ammoniak an einem Katalysator enthaltend Kupfer und TiO₂ (loc. cit., Beispiel 5).

Nachteilig an den Verfahren des Stands der Technik ist, dass bei der aminierenden Hydrierung von Aceton zu geringe Selektivitäten und Ausbeuten erreicht werden. Selbst bei der Verwendung eines hohen molaren Ammoniak-Überschusses bezogen auf Aceton, wie zum Beispiel in GB-A-1218 454, Beispiel 4, wird nur eine Selektivität von maximal 96,24 Gew.-% bezogen auf Aceton erreicht.

Aus der EP-A-514 692 sind Kupfer, Nickel und/oder Kobalt, Zirkonium- und/oder Aluminiumoxid enthaltende Katalysatoren zur katalytischen Aminierung von Alkoholen in der Gasphase mit Ammoniak oder primären Aminen und Wasserstoff bekannt.

Aus der EP-A-382 049 sind Katalysatoren, die sauerstoffhaltige Zirkonium-, Kupfer-, Kobalt- und Nickelverbindungen enthalten, und Verfahren zur hydrierenden Aminierung von Alkoholen oder Carbonylverbindungen bekannt. Der bevorzugte Zirkoniumoxidgehalt dieser Katalysatoren beträgt 70 bis 80 Gew.-% (loc. cit.: Seite 2, letzter Absatz; Seite 3, 3. Absatz; Beispiele).

DE-A-19 53 263 offenbart die Verwendung von Co, Ni und Cu enthaltenden Katalysatoren mit Al₂O₃ oder SiO₂ als Trägermaterial zur Herstellung von Aminen aus Alkoholen.

US 5,952,529 betrifft Aminierungsverfahren unter Verwendung von Katalysatoren umfassend Re, Ni, Co, B und Cu und/oder Ru und einem Trägermaterial.

Die ältere europäische Anmeldung Nr. 99111282.2 vom 10.06.99 betrifft ein Verfahren zur Herstellung von Aminen durch Umsetzung von primären oder sekundären Alkoholen mit Stickstoffverbindungen in Gegenwart von Katalysatoren enthaltend Zirkoniumdioxid, Kupfer, Nickel und Kobalt.

Der vorliegenden Erfindung lag die Aufgabe zugrunde, unter Abhilfe der Nachteile des Stands der Technik die Wirtschaftlichkeit bisheriger Verfahren zur Herstellung von MIPA durch aminierende Hydrierung von Aceton mit Ammoniak zu verbessern. Es sollten Katalysatoren gefunden werden, die einfach zugänglich oder in technisch einfacher Weise herzustellen sind und die es erlauben, die aminierende Hydrierung von Aceton zu MIPA mit hohem Aceton-Umsatz, insbesondere Aceton-Umsätzen von 90 bis 100 %, hoher Ausbeute; hoher Selektivität, insbesondere Selektivitäten von 96,5 bis 100 % (bezogen auf Aceton), und hoher Katalysatorstandzeit bei gleichzeitig hoher mechanischer Stabilität des Katalysatorformkörpers durchzuführen. Die' Katalysatoren sollen demnach eine hohe Aktivität und unter den Reaktionsbedingungen eine hohe chemische Stabilität aufweisen und die obige Aufgabe auch bei Einsatz eines nur geringen molaren Ammoniak-Überschusses bezogen auf Aceton lösen.

Demgemäß wurde ein Verfahren zur Herstellung von Monoisopropylamin (MIPA) durch Umsetzung von Aceton mit Ammoniak und mit Wasserstoff bei erhöhter Temperatur und erhöhtem Druck in Gegenwart eines Katalysators gefunden, welches dadurch gekennzeichnet ist, dass die katalytisch aktive Masse des Katalysators nach dessen Herstellung und vor der Behandlung mit Wasserstoff
40 bis 85 Gew.-% Aluminiumoxid (Al₂O₃),
1 bis 25 Gew.-% sauerstoffhaltige Verbindungen des Kupfers, berechnet als CuO,
5 bis 40 Gew.-% sauerstoffhaltige Verbindungen des Nickels, berechnet als NiO, und
5 bis 40 Gew.-% sauerstoffhaltige Verbindungen des Kobalts, berechnet als CoO, enthält.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper - beispielsweise als Tabletten, Kugeln, Ringe, Extrudate (z. B. Stränge) - in den Reaktor eingebracht werden.

Die Konzentrationsangaben (in Gew.-%) der Komponenten des Katalysators beziehen sich jeweils - falls nicht anders angegeben - auf die katalytisch aktive Masse des hergestellten Katalysators nach dessen letzter Wärmebehandlung und vor der Behandlung mit Wasserstoff.

Die katalytisch aktive Masse des Katalysators, nach dessen letzter Wärmebehandlung und vor der Behandlung mit Wasserstoff, ist als die Summe der Massen der katalytisch aktiven Bestandteile und der Trägermaterialien definiert und enthält im wesentlichen sauerstoffhaltige Verbindungen des Aluminiums, sauerstoffhaltige Verbindungen des Kupfers, sauerstoffhaltige Verbindungen des Nikkels und sauerstoffhaltige Verbindungen des Kobalts.

Die Summe der o.g. katalytisch aktiven Bestandteile und der o.g. Trägermaterialien in der katalytisch aktiven Masse, berechnet als Al₂O₃, CuO, NiO und CoO, beträgt üblicherweise 70 bis 100 Gew.-%, bevorzugt 80 bis 100 Gew.-%, besonders bevorzugt 90 bis 100 Gew.-%, insbesondere 95 bis 100 Gew.-%, ganz besonders 100 Gew.-%.

Die katalytisch aktive Masse der im erfindungsgemäßen. Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

### Beispiele für solche Elemente bzw. deren Verbindungen sind:

Übergangsmetalle, wie Mn bzw. Manganoxide, Re bzw. Rheniumoxide, Cr bzw. Chromoxide, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat, Zink bzw. Zinkoxide, Silber bzw. Silberoxide, Lanthanide, wie Ce bzw. CeO₂ oder Pr bzw. Pr₂O₃, Alkalimetalloxide, wie Na₂O, Alkalimetallcarbonate, wie Na₂CO₃ und K₂CO₃, Erdalkalimetalloxide, wie SrO, Erdalkalimetallcarbonate, wie MgCO₃, CaCO₃, BaCO₃, Phosphorsäureanhydride und Boroxid (B₂O₃).

Die katalytisch aktive Masse der im erfinduingsgemäßen Verfahren eingesetzten Katalysatoren enthält nach dessen Herstellung und vor der Behandlung mit Wasserstoff
40 bis 85 Gew.-%, bevorzugt 50 bis 85 Gew.-%, Aluminiumoxid (Al₂O₃),
1 bis 25 Gew.-%, bevorzugt 1 bis 15 Gew.-%, sauerstoffhaltige Verbindungen des Kupfers, berechnet als CuO,
5 bis 40 Gew.-%, bevorzugt 5 bis 25 Gew.-%, sauerstoffhaltige Verbindungen des Nickels, berechnet als NiO, und
5 bis 40 Gew.-%, bevorzugt 5 bis 25 Gew.-%, sauerstoffhaltige Verbindungen des Kobalts, berechnet als CoO.

Die katalytisch aktive Masse von ganz besonders bevorzugt eingesetzten Katalysatoren enthält nach dessen Herstellung und vor der Behandlung mit Wasserstoff
50 bis 85 Gew.-% Aluminiumoxid (Al₂O₃),
1 bis 15 Gew.-% sauerstoffhaltige Verbindungen des Kupfers, berechnet als CuO,
5 bis 25 Gew.-% sauerstoffhaltige Verbindungen des Nickels, berechnet als NiO, und
5 bis 25 Gew.-% sauerstoffhaltige Verbindungen des Kobalts, berechnet als CoO.

In den im erfindungsgemäßen Verfahren bevorzugt eingesetzten Katalysatoren beträgt das Gewichtsverhältnis von Kobalt zu Nikkel, jeweils berechnet als Metall, 4 : 1 bis 1 :4, besonders bevorzugt 2 : 1 bis 1 : 2.

Weiterhin beträgt in im erfindungsgemäßen Verfahren bevorzugt eingesetzten Katalysatoren das Gewichtsverhältnis von (Kobalt und Nickel) zu Kupfer, jeweils berechnet als Metall, 3 : 1 bis 20 : 1, besonders bevorzugt 3 : 1 bis 10 : 1.

Im erfindungsgemäßen Verfahren bevorzugt eingesetzte Katalysatoren enthalten keine katalytisch aktive Mengen an V, Nb, Ta, Cr, Mo, W, Mn und/oder Re und/oder deren anorganische oder organische Verbindungen.

Darüber hinaus enthalten bevorzugt eingesetzte Katalysatoren keine katalytisch aktive Mengen an Fe, Ru, Rh, Pd, Os, Ir, Pt, Ag und/oder Au und/oder deren anorganische oder organische Verbindungen.

Darüber hinaus enthalten bevorzugt eingesetzte Katalysatoren keine katalytisch aktive Mengen an Zn, In und/oder Sn und/oder deren anorganische oder organische Verbindungen.

Zur Herstellung der Katalysatoren sind verschiedenerlei Verfahrensweisen möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Nickel- Kobalt- und Kupferkomponenten aus einer diese Elemente enthaltenden, wässrigen Salzlösung mittels Mineralbasen in Gegenwart einer Aufschlämmung oder Suspension feinkörniger Pulver einer schwerlöslichen, sauerstoffhaltigen Aluminiumverbindung und anschließendes Waschen, Trocknen und Calcinieren des erhaltenen Präzipitats erhalten werden. Als schwerlösliche, sauerstoffhaltige Aluminiumverbindungen können beispielsweise Aluminiumoxid und Aluminiumoxidhydrat Verwendung finden.

Vorteilhaft werden die im erfindungsgemäßen Verfahren verwendeten Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxidversetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den im erfindungsgemäßen Verfahren verwendeten Katalysatoren weiterverarbeitet. Nach dem Waschen werden sie im allgemeinen bei 80 bis 200°C, vorzugsweise bei 100 bis 150°C, getrocknet und danach calciniert. Die Calcinierung (Wärmebehandlung) wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Tablettenpresse zu Formlingen verpreßt und tempert (wärmebehandelt). Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Bevorzugt werden die im erfindungsgemäßen Verfahren verwendeten Katalysatoren durch Tränkung von Aluminiumoxid (Al₂O₃), das beispielsweise in Form von Pulver, Splitt oder Formkörpern, wie Strängen, Tabletten, Kugeln oder Ringen, vorliegt, hergestellt.

Aluminiumoxid (Al₂O₃) wird beispielsweise in Form von α-, β-, γ-oder θ-Al₂O₃ oder D10-10 von BASF eingesetzt.

Die Herstellung von Formkörpern der o.g. Trägermaterials Aluminiumoxid kann nach den üblichen Verfahren erfolgen.

Die Tränkung dieses Trägermaterials erfolgt ebenfalls nach den üblichen Verfahren, wie z. B. in EP-A-599 180, EP-A-673 918 oder A. B. Stiles, Catalyst Manufacture - Laboratory and Commercial Preparations, Marcel Dekker, Seiten 89 bis 91, New York (1983), beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet (Wärmebehandlung) und gegebenenfalls calziniert.

Die Tränkung kann nach der sogenannten "incipient wetness"-Methode erfolgen, bei der das oxidische Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu kalzinieren. Die mehrstufige Tränkung ist vorteilhaft besonders dann anzuwenden, wenn das Trägermaterial mit einer größeren Metallmenge beaufschlagt werden soll.

Zur Aufbringung mehrerer Metallkomponenten auf das Trägermaterial kann die Tränkung gleichzeitig mit allen Metallsalzen oder in beliebiger Reihenfolge der einzelnen Metallsalze nacheinander erfolgen.

Eine Sonderform der Tränkung stellt die Sprühtrocknung dar, bei der der erwähnte Katalysatorträger in einem Sprühtrockner mit der oder den aufzubringenden Komponente(n) in einem geeigneten Lösungsmittel besprüht wird. Vorteilhaft bei dieser Variante ist die Kombination von Aufbringen und Trocknung der Aktivkomponente (n) in einem Schritt.

Die hergestellten Katalysatoren können als solche gelagert werden. Vor ihrem erfindungsgemäßen Einsatz werden sie üblicherweise durch Behandlung mit Wasserstoff vorreduziert. Sie können jedoch auch ohne diese Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 400°C in einer Wasserstoffatmosphäre behandelt. Bei dieser Vorreduktion wird zumindest ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Das erfindungsgemäße Verfahren läßt sich diskontinuierlich oder bevorzugt kontinuierlich wie folgt durchführen,: wobei der Katalysator bevorzugt als Festbett im Reaktor angeordnet ist. Das Katalysatorbett kann sowohl von oben nach unten als auch von unten nach oben durchströmt werden.

Für die kontinuierliche Verfahrensführung sind Rohrreaktoren besonders geeignet.

Erfindungsgemäß wurde erkannt, dass es bezüglich der Selektivität der Reaktion besonders vorteilhaft ist, die Umsetzung in Reaktoren, insbesondere Festbettreaktoren, auszuführen, bei denen die Reaktionswärme besonders effizient abgeführt werden kann. Dadurch kann eine im wesentlichen gleichbleibende Reaktionstemperatur entlang des Reaktors eingestellt werden.

Daher wird in einer besonders vorteilhaften Ausgestaltung des Verfahrens die Umsetzung von Aceton zu MIPA in einen Rohrbündelreaktor durchgeführt.

Die hydrierende Aminierung des Acetons kann in der Flüssigphase oder in der Gasphase durchgeführt werden.

Üblicherweise arbeitet man bei der Umsetzung bei Temperaturen von 30 bis 300°C, bevorzugt 50 bis 250°C, insbesondere 70 bis 200°C.

Im allgemeinen wird die Reaktion bei einem Absolutdruck von 1 bis 300 bar (0,1 bis 30 MPa) ausgeführt. Bevorzugt werden Absolutdrücke von 5 bis 250 bar, insbesondere von 20 bis 200 bar, angewandt.

Die Anwendung höherer Temperaturen und eines höheren Gesamtdrukkes ist möglich. Der Gesamtdruck im Reaktionsgefäß, welcher sich aus der Summe der Partialdrücke des Ammoniaks, des Acetons, der gebildeten Reaktionsprodukte sowie des gegebenenfalls mitverwendeten Lösungsmittels bei den angegebenen Temperaturen ergibt, wird zweckmäßigerweise durch Aufpressen von Wasserstoff auf den gewünschten Reaktionsdruck eingestellt. Es ist hierbei möglich, statt reinem Wasserstoff ein Gemisch von Wasserstoff und einem Inertgas, wie zum Beispiel Stickstoff, zu verwenden.

Der Wasserstoff wird der Reaktion im allgemeinen in einer Menge von 5 bis 400 Nl, bevorzugt in einer Menge von 50 bis 200 Nl, pro Mol Aceton zugeführt, wobei die Literangaben jeweils auf Normalbedingungen umgerechnet wurden (S.T.P.).

Die Umsetzung erfolgt bevorzugt ohne zusätzliches Lösungsmittel. Ein unter den Reaktionsbedingungen inertes Lösungsmittel, wie Methanol, Ethanol, Propanol, Tetrahydrofuran, Dioxan, N-Methylpyrrolidon, Mihagol oder Ethylenglykoldimethylether, kann jedoch mitverwendet werden.

Je nach Zusammensetzung des erfindungsgemäß verwendeten Katalysators kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Praktisch geht man bei der Durchführung im allgemeinen so vor, dass man dem Katalysator, der sich bevorzugt in einem Rohrbündelreaktor befindet, bei der gewünschten Reaktionstemperatur und dem gewünschten Druck das Aceton und den Ammoniak simultan zuführt. Das molare Verhältnis von Aceton zu Ammoniak beträgt im allgemeinen 1 : 1 bis 1 : 250, bevorzugt 1 : : 1 bis 1 : 50, besonders bevorzugt 1 : 1,2 bis 1 : 10. Dabei belastet man den Katalysator im allgemeinen mit 0,05 bis 5, bevorzugt mit 0,05 bis 2, kg Aceton pro Liter Katalysator (Schüttvolumen) und Stunde. Hierbei ist es zweckmäßig, die Reaktanten bereits vor der Zuführung in das Reaktionsgefäß zu erwärmen.

Der Reaktor kann sowohl in der Sumpf- als auch in der Rieselfahrweise betrieben werden. Überschüssiger Ammoniak kann zusammen mit dem Wasserstoff im Kreis geführt werden.

Aus dem Reaktionsaustrag werden, nachdem dieser zweckmäßigerweise entspannt worden ist, der Ammoniak und der Wasserstoff entfernt und das erhaltene MIPA durch Destillation gereinigt. Zurückgewonnener Ammoniak und Wasserstoff werden vorteilhaft wieder in die Reaktionszone zurückgeführt. Das Gleiche gilt für die eventuell nicht vollständig umgesetztes Aceton oder durch Hydrierung entstandenes Isopropanol. Es ist auch möglich, einen Teil vom Gesamt-Reaktoraustrag zurückzuführen.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der destillativen Aufarbeitung des Reaktionsproduktes aus diesem entfernt.

### Beispiele

### A) Herstellung von Katalysator A (gemäß DE-A-19 53 263)

Aluminiumoxidextrudate mit einem Strangdurchmesser von 4 mm wurden mit einer Lösung überstellt, die jeweils 5 Gew.-% Co und Ni und 2 Gew.-% Cu (berechnet als Metall) enthielt. Es handelte sich hierbei um eine Lösung der Metallnitrate.

Nach ca. 15 Minuten Tränkzeit wurden die Stränge bei 120°C getrocknet und danach bei 520°C getempert.

Danach wurde die Tränkung/Trocknung/Temperung wiederholt.

Der erhaltene Katalysator A hatte die Zusammensetzung:
76 Gew.-% Al₂O₃, 4 Gew.-% Kupferoxid, berechnet als CuO, 10 Gew.-% CoO und 10 Gew.% NiO.

### B) Reaktionsbeispiele 1 bis 12:

Zur kontinuierlichen Versuchsdurchführung wurde ein Hochdruckreaktor mit 45 mm Innendurchmesser und 350 cm Länge verwendet. In der Reaktormitte war in axialer Richtung ein Thermoelement mit 12 mm Durchmesser angebracht. Der Reaktor wurde im unteren Teil mit 700 ml Pallringen aus Edelstahl gefüllt, darüber wurden 3500 ml = 3509 g des Katalysators A in Strangform eingefüllt und als oberste Schüttung wurden abermals 700 ml an Edelstahlpallringen eingefüllt. In den Reaktor wurde von oben nach unten Aceton, Ammoniak und Wasserstoff zugegeben. Der Reaktordruck wurde durch Zufuhr von Wasserstoff eingestellt. Nach dem Reaktor wurde die Reaktionsmischung abgekühlt, auf Normaldruck entspannt und analysiert.

Die detaillierten Versuchsbedingungen und die Ergebnisse sind der folgenden Tabelle zu entnehmen:

| V-Nr. -Nr. | p bar | T °C | Belast.kg/(l*h) | MVNH₃/Ac. | MIPA % (*) | DIPA(*) % (*) | Aceton % (*) | iPrOH% (*) | Sonst. % (*) |
|---|---|---|---|---|---|---|---|---|---|
| 1 | 200 | 150 | 0,1 | 7,8 | 98,9 | 0,05 | 0,01 | 0,9 | 0,1 |
| 2 | 200 | 130 | 0,35 | 2,1 | 96,8 | 0,7 | 0 | 2,2 | 0,3 |
| 3 | 65 | 135 | 0,1 | 4,6 | 97,5 | 0,05 | 0 | 2,1 | 0,3 |
| 4 | 50 | 130 | 0,1 | 6,0 | 97,0 | 0,2 | 0 | 2,2 | 0,6 |
| 5 | 50 | 130 | 0,2 | 3,0 | 97,4 | 0,1 | 0 | 1,7 | 0,8 |
| 6 | 50 | 150 | 0,35 | 2,1 | 97,5 | 0,1 | 0,2 | 1,2 | 0,8 |
| 7 | 50 | 160 | 0,35 | 2,1 | 97,3 | 0,2 | 0,02 | 1,8 | 0,6 |
| 8 | 40 | 135 | 0,1 | 4,6 | 97,5 | 0,05 | 0 | 2,1 | 0,3 |
| 9 | 40 | 140 | 0,2 | 4,6 | 98,1 | 0,02 | 0 | 1,6 | 0,3 |
| 10 | 40 | 140 | 0,4 | 4,6 | 97,8 | 0,02 | 0,3 | 1,3 | 0,4 |
| 11 | 35 | 140 | 0,35 | 2,1 | 97,1 | 0,15 | 0,15 | 1,5 | 1,0 |
| 12 | 35 | 120 | 0,35 | 2,1 | 96,8 | 0,15 | 0,5 | 1,2 | 1,0 |

| | | | | | | | | | |
|---|---|---|---|---|---|---|---|---|---|
| Belast. = Katalysatorbelastung in kg Aceton pro 1 Katalysator (Schüttvolumen) und pro Stunde | | | | | | | | | |
| MV = Molverhältnis | | | | | | | | | |
| Ac. = Aceton | | | | | | | | | |
| DIPA = Diisopropylamin | | | | | | | | | |
| iPrOH = Isopropanol | | | | | | | | | |
| Sonst. = sonstige Nebenprodukte | | | | | | | | | |
| (*) GC-Säule: 30 m Stabilwax DB Amine, 0,25 µm Innendurchmesser, 0,5 µm Filmdicke, | | | | | | | | | |

Temperaturprogramm: 10 Min. bei 50°C, mit 4°C/Min. auf 150°C
Trägergas: Stickstoff
GC-Auswertung: Korrigierte Flächenprozente für MIPA, DIPA und Isopropanol wurden ermittelt, indem zuvor mittels eines Testgemisches aus MIPA + DIPA + iPrOH die GC-Faktoren von DIPA und iPrOH gegenüber MIPA (Faktor 1) bestimmt wurden.

## Patentansprüche

1. Verfahren zur Herstellung von Monoisopropylamin durch Umsetzung von Aceton mit Ammoniak und mit Wasserstoff bei erhöhter Temperatur und erhöhtem Druck in Gegenwart eines Katalysators, **dadurch gekennzeichnet, dass** die katalytisch aktive Masse des Katalysators nach dessen Herstellung und vor der Behandlung mit Wasserstoff
40 bis 85 Gew.-% Aluminiumoxid (Al₂O₃),
1 bis 25 Gew.-% sauerstoffhaltige Verbindungen des Kupfers, berechnet als CuO,
5 bis 40 Gew.-% sauerstoffhaltige Verbindungen des Nickels, berechnet als NiO, und
5 bis 40 Gew.-% sauerstoffhaltige Verbindungen des Kobalts, berechnet als CoO, enthält.

2. Verfahren nach Anspruch 1, **dadurch gekennzeichnet, dass** die katalytisch aktive Masse des Katalysators nach dessen Herstellung und vor der Behandlung mit Wasserstoff
50 bis 85 Gew.-% Aluminiumoxid (Al₂O₃),
1 bis 15 Gew.-% sauerstoffhaltige Verbindungen des Kupfers, berechnet als CuO,
5 bis 25 Gew.-% sauerstoffhaltige Verbindungen des Nickels, berechnet als NiO, und
5 bis 25 Gew.-% sauerstoffhaltige Verbindungen des Kobalts, berechnet als CoO, enthält.

3. Verfahren nach einem der Ansprüche 1 bis 2, **dadurch gekennzeichnet, dass** man die Umsetzung bei Absolutdrücken von 1 bis 300 bar durchführt.

4. Verfahren nach einem der Ansprüche 1 bis 3, **dadurch gekennzeichnet, dass** man die Umsetzung bei Temperaturen von 30 bis 300°C durchführt.

5. Verfahren nach einem der Ansprüche 1 bis 4, **dadurch gekennzeichnet, dass** man den Katalysator in Form von Formkörpern einsetzt.

6. Verfahren nach einem der Ansprüche 1 bis 5, **dadurch gekennzeichnet, dass** man die Umsetzung in einem Rohrbündelreaktor durchführt.

7. Verfahren nach einem der Ansprüche 1 bis 6, **dadurch gekennzeichnet, dass** man die Umsetzung in der Gasphase durchführt.

8. Verfahren nach einem der Ansprüche 1 bis 7, **dadurch gekennzeichnet, dass** man ein Teil vom Gesamt-Reaktoraustrag zurückführt.

## Claims

1. A process for preparing monoisopropylamine by reacting acetone with ammonia and with hydrogen at elevated temperature and elevated pressure in the presence of a catalyst, wherein the catalytically active mass of the catalyst comprises, after its preparation and before the treatment with hydrogen,
from 40 to 85% by weight of aluminum oxide (Al₂O₃),
from 1 to 25% by weight of oxygen-containing compounds of copper, calculated as CuO,
from 5 to 40% by weight of oxygen-containing compounds of nickel, calculated as NiO, and
from 5 to 40% by weight of oxygen-containing compounds of cobalt, calculated as CoO.

2. A process as claimed in claim 1, wherein the catalytically active mass of the catalyst comprises, after its preparation and before the treatment with hydrogen,
from 50 to 85% by weight of aluminum oxide (Al₂O₃),
from 1 to 15% by weight of oxygen-containing compounds of copper, calculated as CuO,
from 5 to 25% by weight of oxygen-containing compounds of nickel, calculated as NiO, and
from 5 to 25% by weight of oxygen-containing compounds of cobalt, calculated as CoO.

3. A process as claimed in either of claims 1 and 2, wherein the reaction is carried out under absolute pressures of from 1 to 300 bar.

4. A process as claimed in any of claims 1 to 3, wherein the reaction is carried out at temperatures of from 30 to 300°C.

5. A process as claimed in any of claims 1 to 4, wherein the catalyst is employed in the form of shaped articles.

6. A process as claimed in any of claims 1 to 5, wherein the reaction is carried out in a tube bundle reactor.

7. A process as claimed in any of claims 1 to 6, wherein the reaction is carried out in the gas phase.

8. A process as claimed in any of claims 1 to 7, wherein part of the total discharge from the reactor is returned.

## Revendications

1. Procédé de préparation de monoisopropylamine au moyen de la réaction de l'acétone avec de l'ammoniac et de l'hydrogène sous une température et une pression élevées en présence d'un catalyseur, **caractérisé en ce que** la masse à activité catalytique contient, après sa préparation et avant traitement avec de l'hydrogène,
40 à 85 % en poids d'oxyde d'aluminium (Al₂O₃),
1 à 25 % en poids de composés oxygénés du cuivre, exprimé en CuO,
5 à 40 % en poids de composés oxygénés du nickel, exprimé en NiO, et
5 à 40 % en poids de composés oxygénés du cobalt, exprimé en CoO.

2. Procédé selon la revendication 1, **caractérisé en ce que** la masse à activité catalytique contient, après sa préparation et avant traitement avec de l'hydrogène,
50 à 85 % en poids d'oxyde d'aluminium (Al₂O₃),
1 à 15 % en poids de composés oxygénés du cuivre, exprimé en CuO,
5 à 25 % en poids de composés oxygénés du nickel, exprimé en NiO, et
5 à 25 % en poids de composés oxygénés du cobalt, exprimé en tant que CoO.

3. Procédé selon la revendication 1 ou 2, **caractérisé en ce que** l'on procède à la réaction sous des pressions absolues allant de 1 bar à 300 bars.

4. Procédé selon l'une quelconque des revendications 1 à 3, **caractérisé en ce que** l'on procède à la réaction à des températures allant de 30 °C à 300°C.

5. Procédé selon l'une quelconque des revendications 1 à 4, **caractérisé en ce que** l'on met en oeuvre le catalyseur sous la forme de corps moulés.

6. Procédé selon l'une quelconque des revendications 1 à 5, **caractérisé en ce que** l'on procède à la réaction dans un réacteur à faisceaux tubulaires.

7. Procédé selon l'une quelconque des revendications 1 à 6, **caractérisé en ce que** l'on procède à la réaction en phase gazeuse.

8. Procédé selon l'une quelconque des revendications 1 à 7, **caractérisé en ce que** l'on recycle une partie de la totalité du produit du réacteur.
